(19) Europäisches Patentamt
European Patent Office
Office européen des brevets

(11) EP 1 277 461 A2

(12) **EUROPÄISCHE PATENTANMELDUNG**

(43) Veröffentlichungstag:
**22.01.2003 Patentblatt 2003/04**

(51) Int Cl.[7]: **A61K 7/42**

(21) Anmeldenummer: **02016101.4**

(22) Anmeldetag: **19.07.2002**

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **20.07.2001 DE 10135518**

(71) Anmelder: **Beiersdorf AG**
**20245 Hamburg (DE)**

(72) Erfinder:
- **Göppel, Anja**
  **22527 Hamburg (DE)**
- **Eitrich, Anja**
  **22587 Hamburg (DE)**

(54) **Kosmetische Formulierungen mit Triazinen als Lichtschutzfilter sowie Dihydroxyaceton (DHA)**

(57) Die Erfindung betrifft kosmetische Formulierungen, welche mindestens einen UV-Filter aus der Substanzklasse der Triazine, mindestens eine Selbstbräunungssubstanz (vorzugsweise 1,3-Dihydroxyaceton) enthalten und frei sind von Dibenzoylmethan- und Campherderivaten.

EP 1 277 461 A2

**Beschreibung**

Die vorliegende Erfindung betrifft kosmetische Formulierungen, die UV-Filter aus der Gruppe der Triazine, mindestens eine Selbstbräunungssubstanz (z.B.: 1,3-Dihydroxyaceton) enthalten und frei von Dibenzoylmethan- und Campherderivaten sind.

Die vorliegende Erfindung betrifft kosmetische Formulierungen die Lichtschutzfilter und Selbstbräuner enthalten.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.
Zum Schutz der Haut wurden daher eine Reihe von Lichtschutzfiltersubstanzen entwickelt, die in kosmetischen Zubereitungen eingesetzt werden können. Diese UVAund UVB-Filter sind in den meisten Industrieländern in Form von Positivlisten wie der Anlage 7 der Kosmetikverordnung zusammengefasst.

Zu den eingesetzten UV-Lichtschutzfiltern zählt unter anderem 2-Hydroxy-4-methoxybenzophenon. Dieser UV-Breitbandfilter ist unter den Handelsnamen Eusolex 4360 (Merck), Neo Heliopan BB (H&R), Escalol 567 (ISP) und Uvasorb MET/C (Sigma)

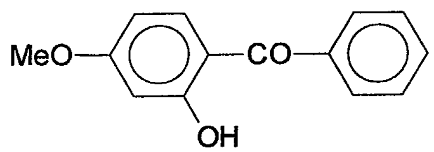

kommerziell erhältlich.
Desweiteren wird 1-(4-*tert*.-Butylphenyl)-3-(4'-methoxyphenyl)-propan-1,3-dion als UVA-Schutzfilter eingesetzt [Eusolex 9020 (Merck), Parsol 1789 (Givaudan)].

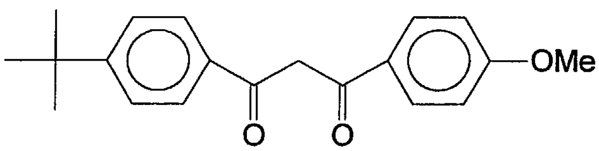

Weitere in der Praxis verwendete Sonnenschutzfilter sind unter anderem 4-Methoxyzimtsäure-2-ethylhexylester [Escalol 557 (ISP), Eusolex 2292 (Merck), Neo Heliopan AV (H&R), Parsol MCX (Givaudan)]

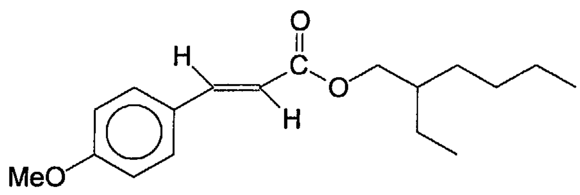

sowie 3-(4'-Methyl)benzyliden-bornan-2-on [Eusolex 6300(Merck), Neo Heliopan MBC (H&R), Parsol 5000 (Givaudan)].

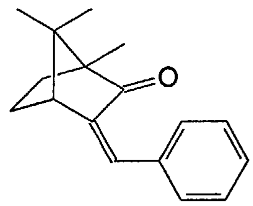

Eine weitere Klasse an UV-Lichtschutzfiltern leitet sich von den Benzimidazolsulfonsäuren ab.

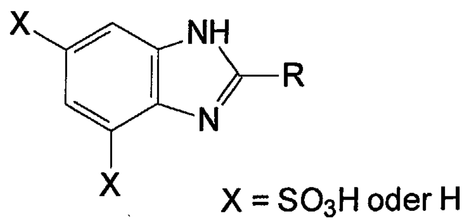

$X = SO_3H$ oder H

Hierzu zählen die 2-Phenylbenzimidazol-5-sulfonsäure und ihre Natrium-, Kalium- und Triethanolammoniumsalze [Eusolex 232 (Merck), Neo Heliopan Hydro (H&R), Parsol HS (Givaudan)]

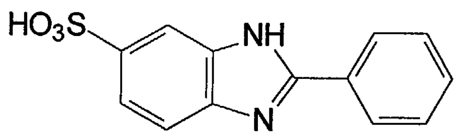

sowie das Bisimidazolidat Phenylen-1,4-bis-(2-benzimidazyl)-3,3',5,5'-tetrasulfonsäure und ihre Natrium-, Kalium- und Triethanolammoiumsalze, insbesondere das Di-Natriumsalz.

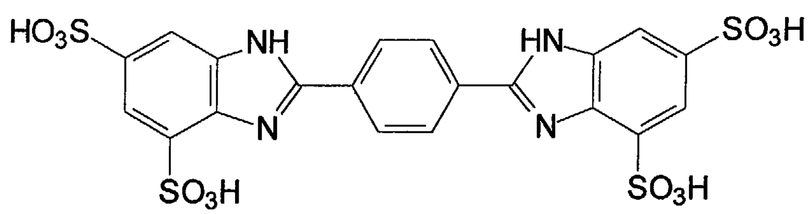

Eine weitere Klasse von Lichtschutzfiltern, leitet sich von dem Strukturelement des Benzotriazols ab:

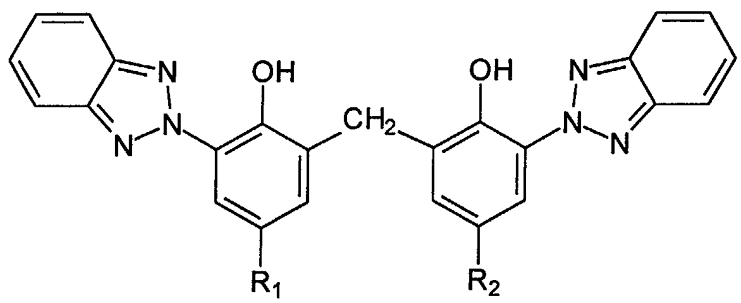

$R_1$ und $R_2$ können unabhängig voneinander aus der Gruppe der verzweigten oder unverzweigten $C_1$-$C_{18}$-Alkylreste gewählt werden, die gegebenenfalls mit einer oder mehreren $C_1$-$C_4$- Alkylgruppen, $C_5$-$C_{12}$-Cycloalkyl- oder Arylresten substituiert sind.

Hierzu gehört das 2,2'-Methylen-bis-(6-(2H-benzotriazol-2-yl)-4-(1,1,3,3-tetramethylbutyl)-phenol), welches durch die chemische Strukturformel

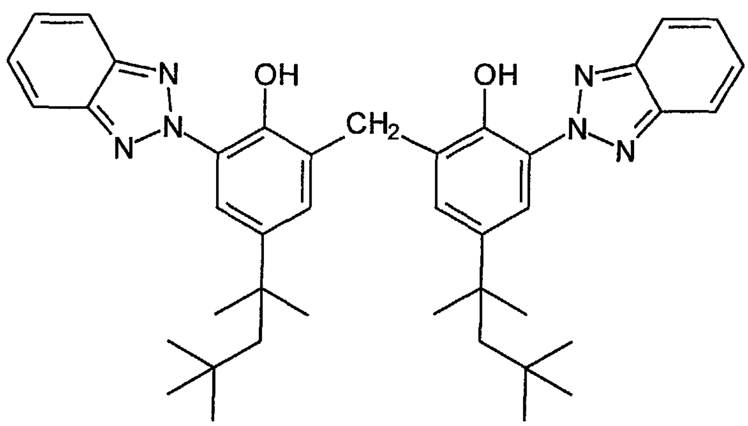

gekennzeichnet ist.

Auch Monomere Benzotriazole wie der Breitbandfilter 2-(2H-benzotriazol-2-yl)-4-methyl-6-[2-methyl-3-[1,3,3,3 -tetramethyl-1-[(trimethylsilyl)oxy]disiloxanyl]propyl]-phenol (CAS-Nr.: 155633-54-8) mit der INCI-Bezeichnung Drometrizole Trisiloxane

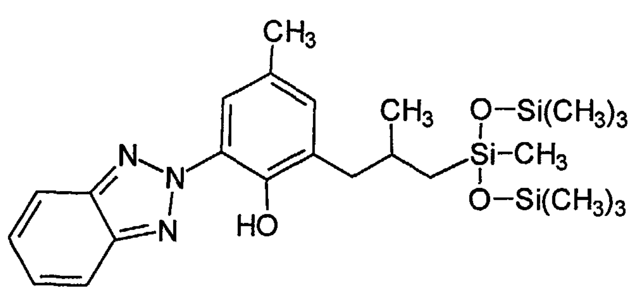

werden eingesetzt.

Eine weitere Wichtige Klasse von UV-Filtern leitet sich vom Triazin ab:

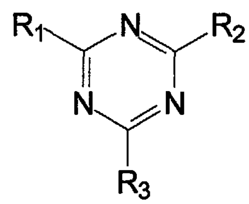

Den natürlichen Schutz vor den negativen Folgen der Sonnenstrahlung bietet die Bräunung (Pigmentierung) der Haut. Die Epidermis enthält in ihrer untersten Schicht, der Basalschicht, neben den Basalzellen einzelne pigmentbildende Zellen, die Melanocyten. Durch UV-Licht wird in diesen Zellen die Produktion von Melanin angeregt, das in die Kerantionocycten (Hornzellen) transportiert und dort als braune Hautfarbe sichtbar wird.

Diese von der Aminosäure Tyrosin ausgehende Pigmentbildung wird überwiegend durch UVB-Strahlung initiiert und als "indirekte Pigmentierung" bezeichnet. Ihre Entwicklung läuft über mehrere Tage; die so erhaltene Sonnenbräune besteht über einige Wochen.
Bei der "Direkt-Pigmentierung", die mit der Sonnenbestrahlung einsetzt, werden vorwiegend farblose Melanin-Vorstufen durch UVA-Strahlung zu dunkel gefärbten Melanin oxidiert. Da diese Oxidierung reversibel ist, führt sie zu einer nur kurz anhaltenden Hautbräunung.

Eine künstliche Bräunung der Haut lässt sich äußerlich mit Hilfe von Schminke und oral durch Einnahme von Carotinoiden erzeugen.

Weitaus beliebter jedoch ist die künstliche Bräunung der Haut, welche sich durch Auftragen von sogenannten Selbstbräunern erzielen lässt. Diese Verbindungen weisen als chemisches Strukturmerkmal Keto- bzw. Aldehydgruppen in Nachbarschaft zu Alkoholfunktionen auf. Diese Ketole bzw. Aldole gehören überwiegend zur Substanzklasse der Zucker. Ein besonders häufig eingesetzter Selbstbräuner ist 1,3-Dihydroxyaceton.
Die Verbindungen können mit den Proteinen und Aminosäuren der Hornschicht der Haut im Sinne einer Maillard-Reaktion umgesetzt werden, wobei über einen noch nicht vollständig aufgeklärten Reaktionsweg Polymerisate entstehen, die der Haut einen bräunlichen Farbton verleihen. Diese Reaktion ist nach etwa 4 bis 6 Stunden abgeschlossen; die so erzielte Bräune ist nicht abwaschbar und wird erst mit der normalen Hautabschuppung entfernt.

Im Gegensatz zur natürlichen Pigment-Bräunung (Sonnenbräunung) bietet die mittels Selbstbräuner erzielte Bräunung jedoch keinerlei Schutz vor UV-Strahlung. Ein weiterer Nachteil von 1,3-Dihydroxyaceton besteht darin, dass es, insbesondere unter dem Einfluss ultravioletter Strahlung, wenn auch in meist geringen Mengen Formaldehyd abspaltet. Generell neigt 1,3-Dihydroaceton in Formulierungen zur Zersetzung.
Um die kosmetische Formulierungen auf Basis von 1,3-Dihydroxyaceton und lipophilen UV-Filtern zu stabilisieren werden Dibenzoylmethan- und Campherderivate als UV-Schutzfilter verwendet. Darüber hinaus können sie auch Filter auf Triazinbasis enthalten.

Bei den Inhaltsstoffen kosmetischer Formulierungen besteht immer ein Restrisiko, dass diese Allergien auslösen können. Insbesondere besteht diese Gefahr bei UV-Schutzfiltern, da sie einer intensiven Energiezufuhr ausgesetzt sind. Bei der Bestrahlung mit Sonnenlicht entstehen zu einem geringen Teil labile Verbindungen über deren Penetrationsfähigkeit und Toxizität wenig bekannt ist. Diese können gegebenenfalls Photoallergien auslösen. Insbesondere trifft das auf Verbindungen wie 4-Isopropyl-dibenzoylmethan zu. Auch das verwandte 1-(4-tert.-Butylphenyl)-3-(4'methoxyphenyl)propan-1,3-dionderivat zersetzt sich unter UV-Lichteinwirkung und muss mit Campherderivaten wie 3-(4'-Methyl)benzylidenbornan-2-on stabilisiert werden. Diese Campherderivate (z.B. 3-(4'-Methyl)benzylidenbornan-2-on) sind jedoch im Vergleich zu anderen UV-Filtern nicht besonders verträglich. Eine Übersicht über das Gefährdungspotential von UV-Filtern bietet die "Göttinger Liste" von S. Schrader und Mitarbeitern.
Es war daher das Ziel der vorliegenden Erfindung, die Risiken, welche von den in kosmetischen Formulierungen eingesetzten UV-Filtern ausgehen, weiter zu verringern.

Vollkommen überraschend und für den Fachmann nicht voraussehbar gelingt es, die Anzahl der UV-Filter in einer 1,3-Dihydroxyaceton enthaltenen kosmetischen Formulierung zu reduzieren und gleichzeitig die Stabilität des sich leicht zersetzenden Selbstbräuners zu erhöhen. Auf diese Weise lassen sich selbstbräunende, photostabile und vor UVA- und UVB-Strahlung schützende kosmetische Formulierungen darstellen, deren Hautverträglichkeit erhöht ist. Zwar beschreibt die DE 19949826 Wirkstoffkombinationen aus 1,3-Dihydroxyaceton, Verbindungen mit dem Strukturmotiv des Dibenzoylmethans, des Methylencamphers und gewünschtenfalls Triazinen. Doch konnte sie nicht den Weg zu dieser Erfindung bahnen, da Dibenzoylmethan- und Methylencampherderivate eine elementare Vorraussetzung für die Stabilisierung des DHA darstellten.
Insbesondere wurde die Aufgabe, auf die aus allergologischer Sicht bedenklichen Dibenzoylmethanderivate (z.B. 4-Isopropyl-dibenzoylmethan, Butyl Methoxydibenzoyimethan ) zu verzichten und damit die Produktverträglichkeit und Photostabilität entscheidend zu erhöhen, durch die vorliegende Erfindung gelöst. Erstmalig können auf diese Weise

stabile kosmetische Formulierungen mit dem Selbstbräuner 1,3-Dihydroxyaceton hergestellt werden, die frei von Dibenzoylmethan- und Campherderivaten sind und Triazine als öllösliche UV-Schutzfilter enthalten. Dabei bedeutet frei von Dibenzoylmethan- und Campherderivaten, dass die erfindungsgemäßen kosmetischen Formulierungen höchstens 0,5 Gewichts-%, bevorzugt höchstens 0,1 Gewichts-% (jeweils bezogen auf das Gesamtgewicht der Formulierung) und besonders bevorzugt überhaupt keine Dibenzoylmethan- und Campherderivate enthalten.

Als vorteilhafte Selbstbräuner können erfindungsgemäß unter anderem eingesetzt werden:

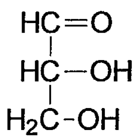

Glycerolaldehyd

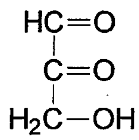

Hydroxymethylglyoxal

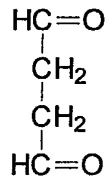

γ-Dialdehyd

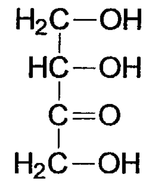

Erythrulose

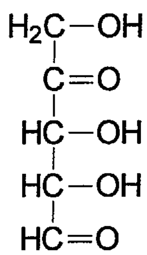

6-Aldo-D-Fructose

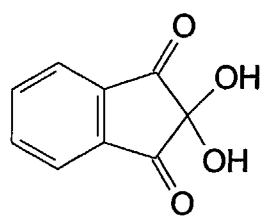

Ninhydrin

Ferner ist das 5-Hydroxy-1,4-naphtochinon (Juglon) zu nennen, das aus den Schalen frischer Walnüsse extrahiert wird

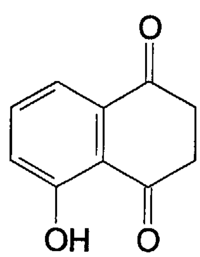

5-Hydroxy-1,4-naphtochinon (Juglon)

sowie das in den Henna-Blättern vorkommende 2-Hydroxy-1,4-naphtochinon (Lawson).

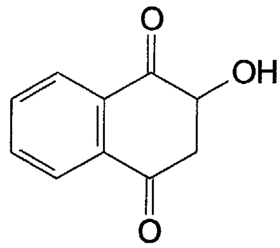

2-Hydroxy-1,4-naphtochinon (Lawson)

Ganz besonders bevorzugt im Sinne der Erfindung ist das 1,3-Dihydroxyaceton (DHA), ein im menschlichen Körper vorkommender dreiwertiger Zucker.

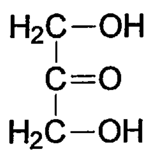

1,3-Dihydroxyaceton (DHA)

Als UV-Filter aus der Reihe der Triazine kommen unter anderem in Frage der symmetrisch substituierte 4,4',4"-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin. [UVINUL T 150 (BASF)].

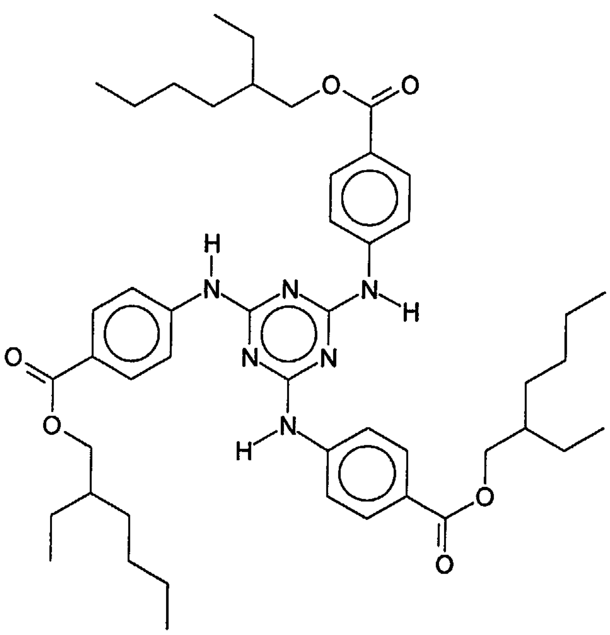

Ferner können unsymmetrisch substituierte s-Triazinderivate verwendet werden, beispielsweise solche, wie sie in der EP-A-570 838 beschrieben werden, deren chemische Struktur durch die generische Formel

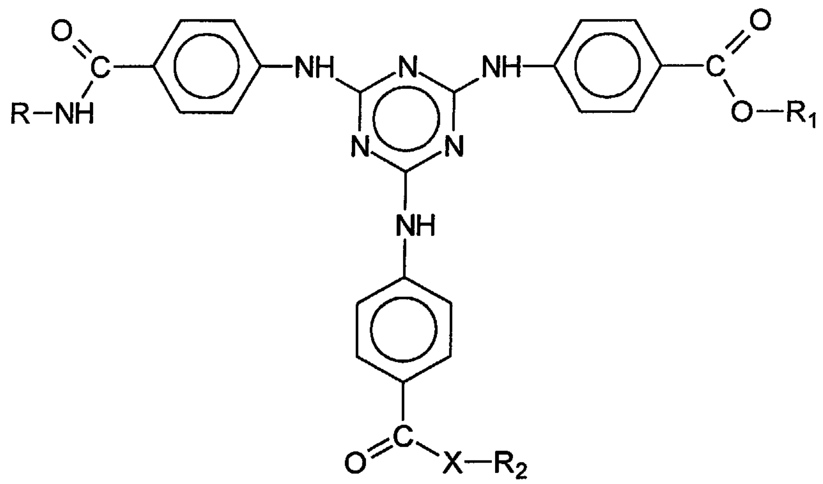

wiedergegeben wird, wobei

- R einen verzweigten oder unverzweigten $C_1$ - $C_{18}$-Alkylrest, einen $C_5$-$C_{12}$-Cycloalkylrest, gegebenenfalls substituiert mit einer oder mehreren $C_1$ - $C_4$ - Alkylgruppen, darstellt,
- X ein Sauerstoffatom oder eine NH-Gruppe darstellt,
- $R_1$ einen verzweigten oder unverzweigten $C_1$ - $C_{18}$-Alkylrest, einen $C_5$ - $C_{12}$-Cycloalkylrest, gegebenenfalls substituiert mit einer oder mehreren $C_1$ - $C_4$ - Alkylgruppen, oder ein Wasserstoffatom, ein Alkalimetallatom, eine Ammoniumgruppe oder eine Gruppe der Formel

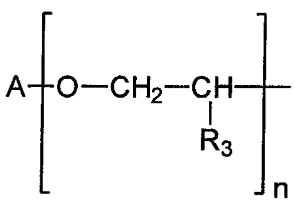

bedeutet, in welcher

- A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$-Alkylgruppen,
- $R_3$ ein Wasserstoffatom oder eine Methylgruppe darstellt,
- n eine Zahl von 1 bis 10 darstellt,

- $R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$-Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$-Alkylgruppen, oder ein Wasserstoffatom, ein Alkalimetallatom, eine Ammoniumgruppe oder eine Gruppe der Formel

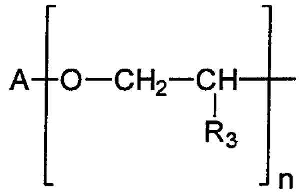

bedeutet, in welcher

A einen verzweigten oder unverzweigten $C_1$- $C_{18}$-Alkylrest, einen $C_5$-$C_{12}$-Cycloalkyl- oder Arylrest darstellt, gegebenenfalls substituiert mit einer oder mehreren $C_1$ - $C_4$- Alkylgruppen,

$R_3$ ein Wasserstoffatom oder eine Methylgruppe darstellt,

n eine Zahl von 1 bis 10 darstellt, wenn X ein Sauerstoffatom darstellt.

Insbesondere vorteilhaft sind solche unsymmetrisch substituierte s-Triazine, aus der Gruppe der Substanzen gewählt, welche in der EP-A-775 698 beschrieben werden:

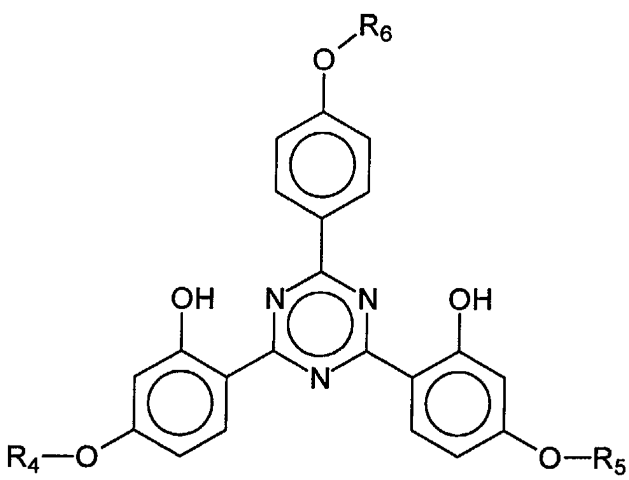

und/oder

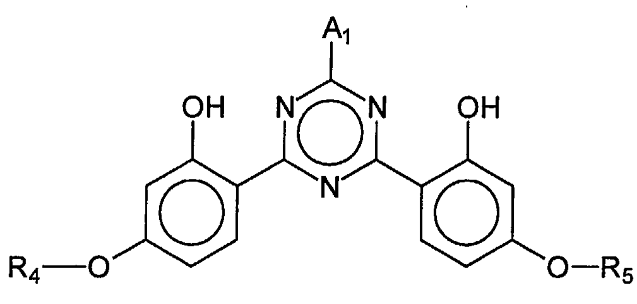

Alle in dieser Schrift erwähnten Bis-Resorcinyltriazine, seien sie durch generische oder durch konkrete Formeln offenbart, sind vorteilhaft im Sinne der vorliegenden Erfindung. Ganz besonders vorteilhaft werden $R_4$ und $R_5$ aus der Gruppe der verzweigten und unverzweigten Alkylgruppen von 1 bis 18 Kohlenstoffatomen gewählt. Auch können die Alkylgruppen wiederum vorteilhaft mit Silyloxygruppen substituiert sein.

$A_1$ stellt vorteilhaft einen substituierten homo- oder heterocyclischen aromatischen Fünfring oder Sechsring dar.

Ganz besonders vorteilhaft sind folgende Verbindungen:

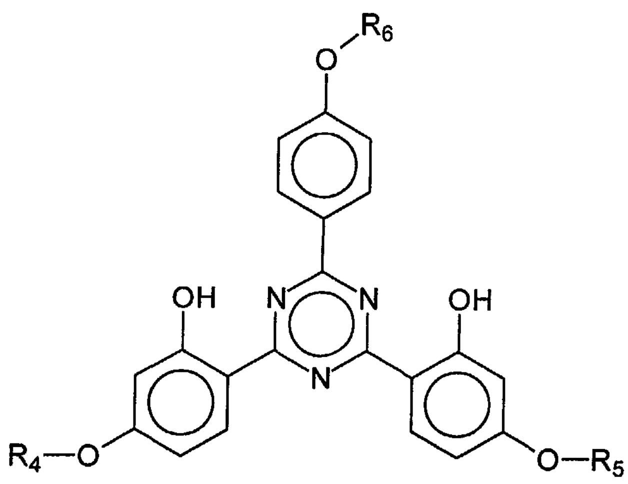

wobei $R_6$ ein Wasserstoffatom oder eine verzweigte oder unverzweigte Alkylgruppe mit 1 bis 10 Kohlenstoffatomen darstellt, insbesondere das 2,4-Bis-{[4-(2-ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich und durch folgende Struktur gekennzeichnet ist:

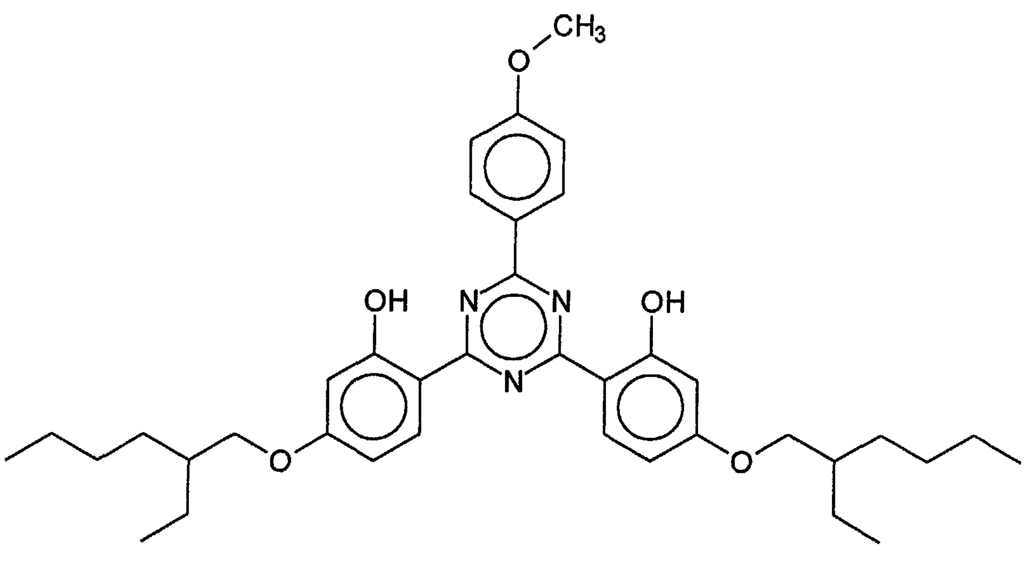

Es ist auch gegebenenfalls vorteilhaft, Zubereitungen gemäß der Erfindung mit einem Gehalt an 2,4-Bis-{[4-(3-sulfonato)-2-hydroxy-propyloxy)-2-hydroxy]-phenyl}-6-(4-methoxyphenyl)-1,3,5-triazin Natriumsalz zu versehen, welches durch folgende Struktur gekennzeichnet ist:

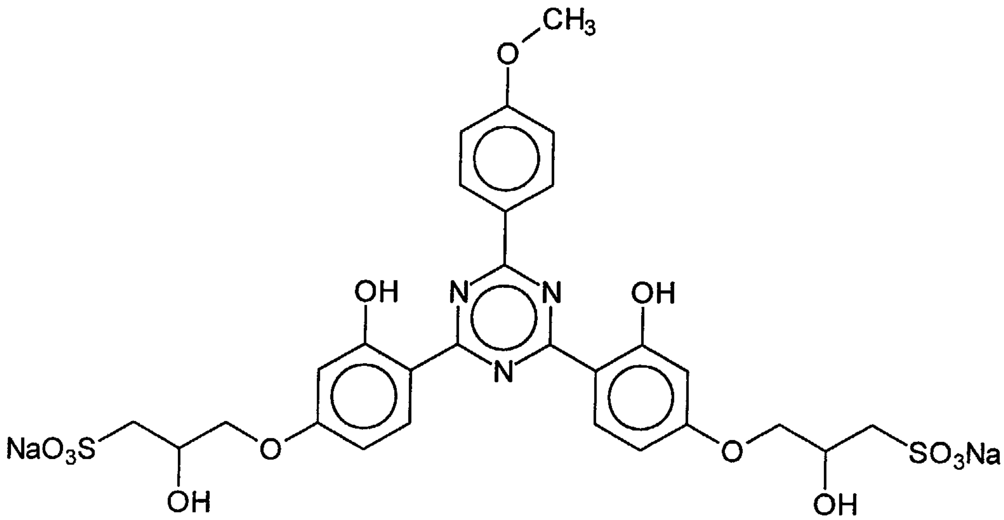

Auch ist es vorteilhaft, Zubereitungen gemäß der Erfindung mit 2,4-Bis-{[4-(3-(2-propyloxy)-2-hydroxy-propyloxy)-2-hydroxy]-phenyl}-6-(4-methoxyphenyl)-1,3,5-triazin zu versehen, welches durch folgende Struktur gekennzeichnet ist:

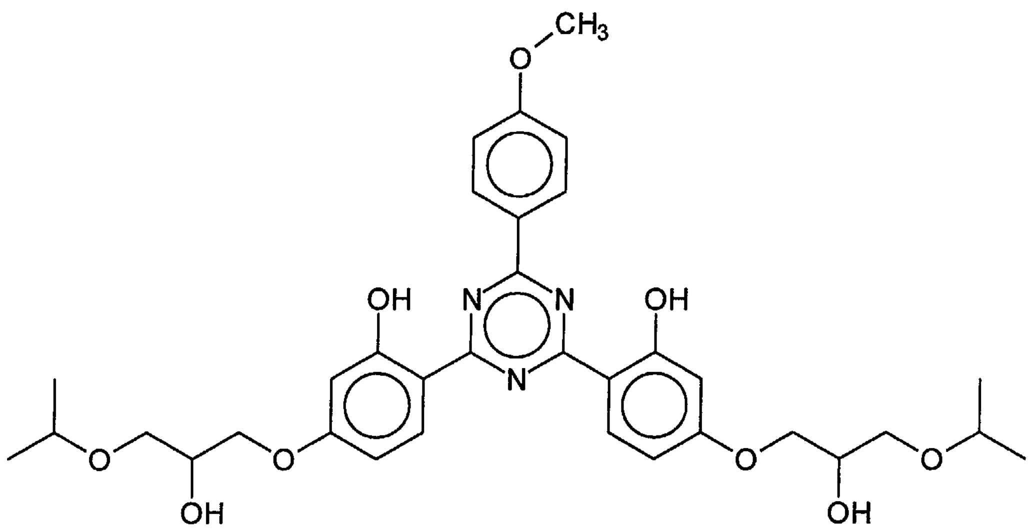

Ferner ist es erfindungsgemäß von Vorteil 2,4-Bis-{[4-(2-ethyl-hexyloxy)-2-hydroxy]-phenyl}-6-[4-(2-methoxyethyl-carboxyl)-phenylamino]-1,3,5-triazin als UV-Filter zu verwenden, welches durch folgende Struktur gekennzeichnet ist:

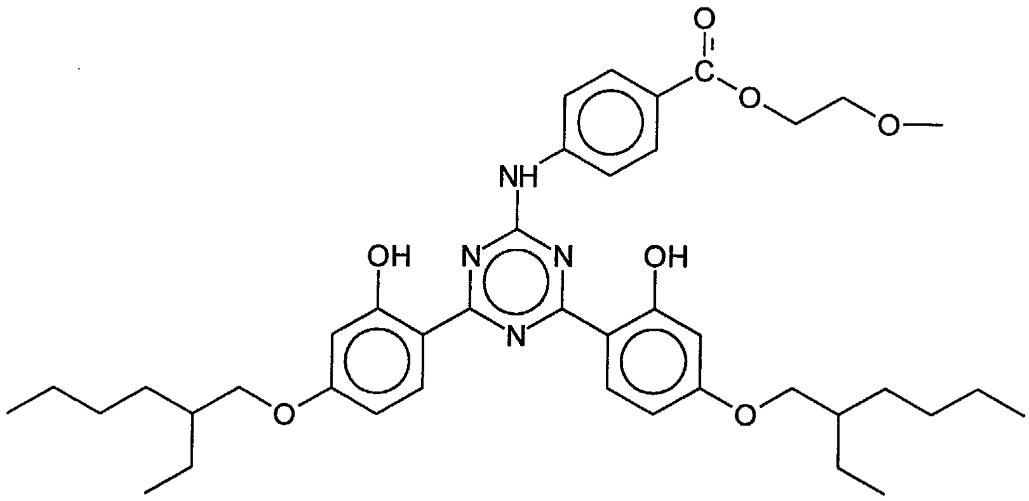

Weiterhin ist 2,4-Bis-{[4-(3-(2-propyloxy)-2-hydroxy-propyloxy)-2-hydroxy]-phenyl}-6-[4-(ethylcarboxyl)-phenylamino]-1,3,5-triazin erfindungsgemäß von Vorteil. Es wird durch folgende Struktur beschrieben:

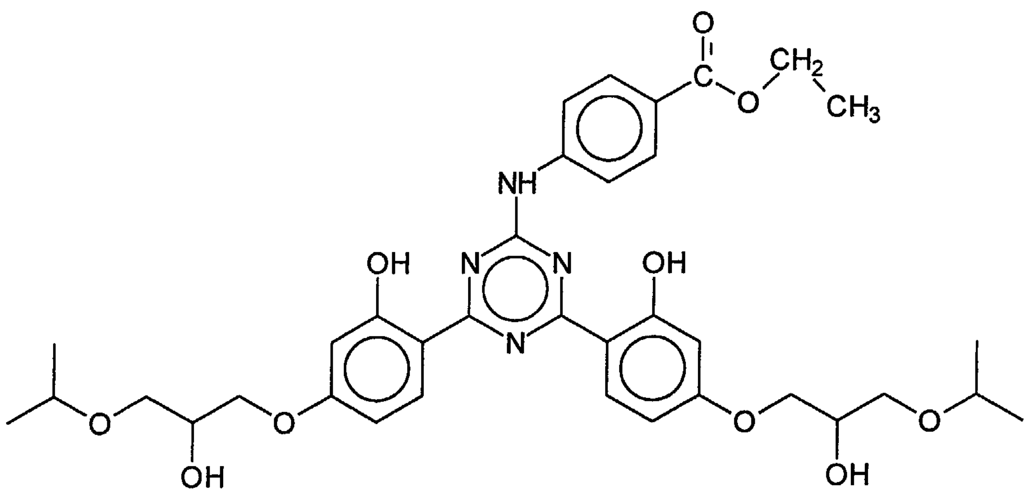

Ferner kann 2,4-Bis-{[4-(2-ethyl-hexyloxy)-2-hydroxy]-phenyl}-6-(1-methyl-pyrrol-2-yl)-1,3,5-triazin im Sinne der Erfindung verwendet werden, welches durch folgende Struktur gekennzeichnet ist:

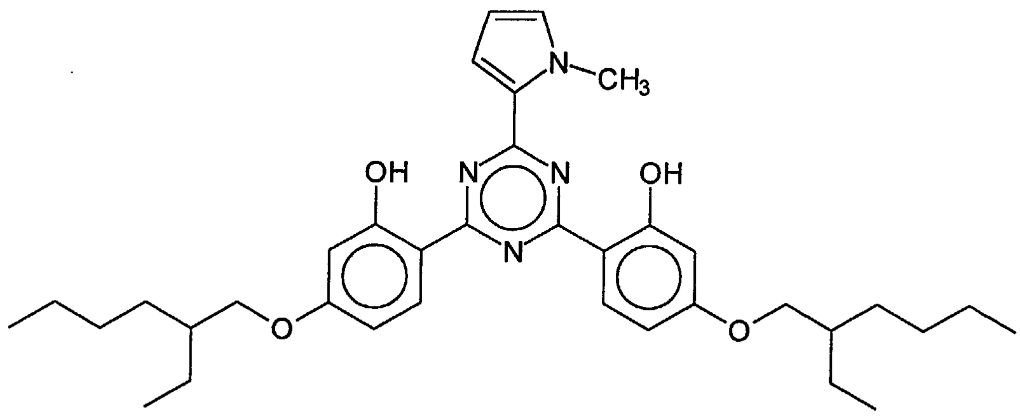

Weitere im Sinne der Erfindung vorteilhafte UV-Filer aus der Gruppe der Triazine sind 2,4-Bis-{[4-tris(trimethylsiloxy-silylpropyloxy)-2-hydroxy]-phenyl}-6-(4-methoxyphenyl)-1,3,5-triazin, welches durch folgende Struktur gekennzeichnet ist,

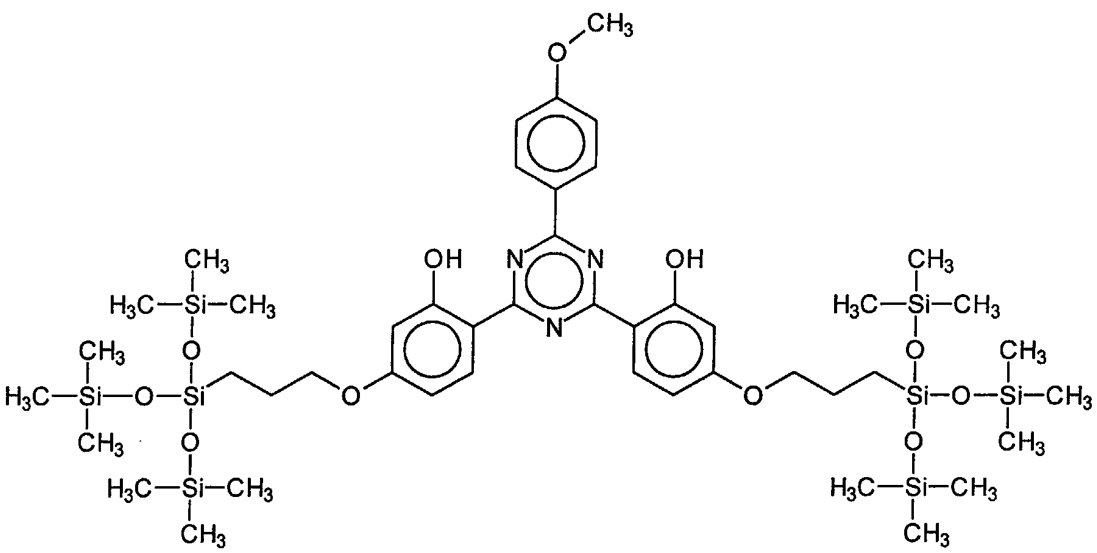

sowie 2,4-Bis-{[4-(2-methylpropenyloxy)-2-hydroxy]-phenyl}-6-(4-methoxyphenyl)-1,3,5-triazin zu versehen, welches durch folgende Struktur gekennzeichnet ist:

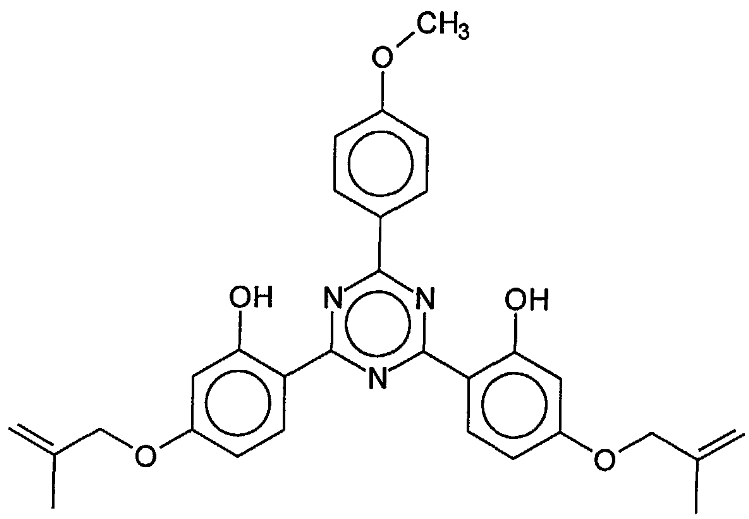

Ferner gehören zu den erfindungsgemäß vorteilhaften UV-Filtern 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin zu versehen, welches durch folgende Struktur gekennzeichnet ist:

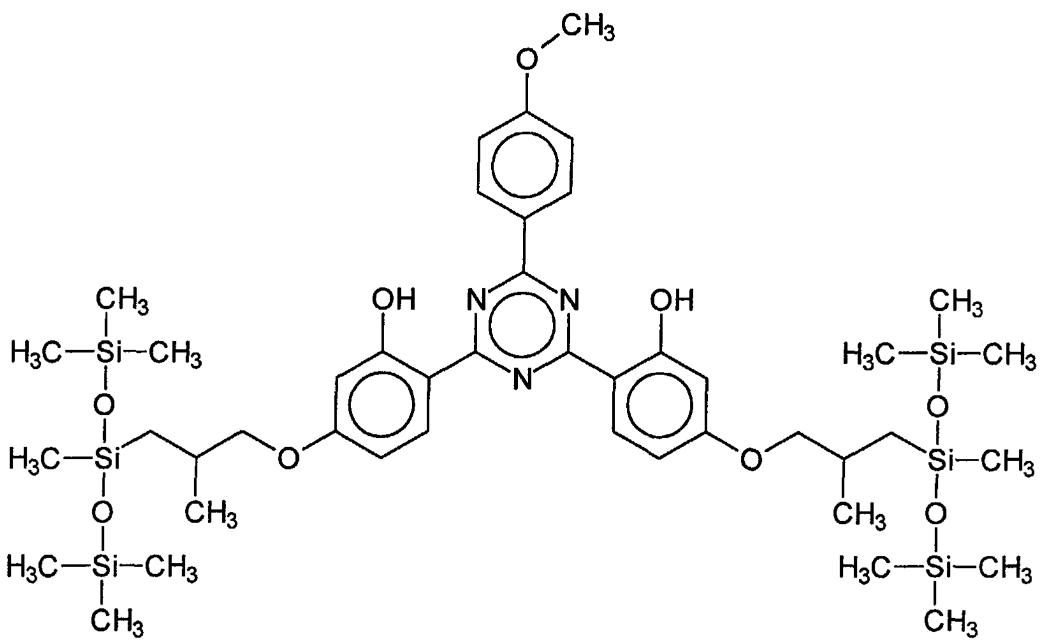

sowie das asymmetrisch substituierte s-Triazin, dessen chemische Struktur durch die Formel

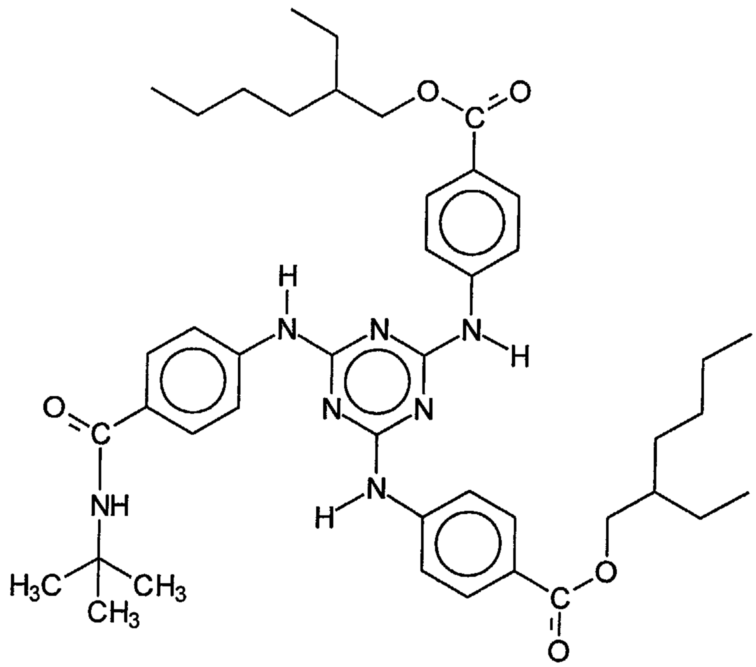

wiedergegeben wird, welches auch als Dioctylbutylamidotriazon (INCI: Dioctylbutamidotriazone) bezeichnet wird und unter der Handelsbezeichnung UVASORB HEB bei Sigma 3V erhältlich ist.

Die Liste der genannten Selbstbräuner und UV-Filter, die in den erfindungsgemäßen Zubereitungen verwendet werden können, soll selbstverständlich nicht limitierend sein.

Auch können erfindungsgemäß mehrere UV-Filter und/oder mehrere Selbstbräuner miteinander kombiniert werden.

Es ist vorteilhaft im Sinne der Erfindung die Selbstbräunungssubstanzen in einer Konzentration von 0,1% bis 15% insbesondere von 0,2% bis 10% einzusetzen.

Der im Sinne der Erfindung vorteilhafte Konzentrationsbereich für 1,3-Dihydroxyaceton liegt bei 0,5 -10 Gewichts-%.

Für die Konzentration an UV-Filtern wird vorzugsweise ein Anteil von 0,01% bis 15% und besonders vorteilhaft der Konzentrationsbereich von 0,1% bis 10% gewählt.

Das Verhältnis von UV-Filter zu Selbstbräuner liegt vorzugsweise bei 0,1:10 bis 10 : 0,1.

Erfindungsgemäß können die kosmetischen und/oder dermatologischen Lichtschutzformulierungen wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter enthalten.

Kosmetische und dermatologische Zubereitungen gemäß der Erfindung enthalten vorteilhaft, wenngleich nicht zwingend, anorganische Pigmente, welche röntgenamorph oder nichtröntgenamorph sind, auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Röntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente durch Flammenreaktion erhältlich, beispielsweise dadurch, daß ein Metall- oder Halbmetallhalogenid mit Wasserstoff und Luft (oder reinem Sauerstoff) in einer Flamme umgesetzt wird.

In kosmetischen, dermatologischen oder pharmazeutischen Formulierungen werden röntgenamorphe Oxidpigmente als Verdickungs- und Thixotropierungsmittel, Fließhilfsmittel, zur Emulsions- und Dispersionsstabilisierung und als Trägersubstanz (beispielsweise zur Volumenerhöhung von feinteiligen Pulvern oder Pudern) eingesetzt.

Bekannte und in der kosmetischen oder dermatologischen Galenik oftmals verwendete röntgenamorphe Oxidpigmente sind die Siliciumoxide des Typs Aerosil® (CAS-Nr. 7631-86-9. Aerosile®, erhältlich von der Gesellschaft

DEGUSSA, zeichnen sich durch geringe Partikelgröße (z.B. zwischen 5 und 40 nm) aus, wobei die Partikel als kugelförmige Teilchen sehr einheitlicher Abmessung anzusehen sind. Makroskopisch sind Aerosile® als lockere, weiße Pulver erkenntlich. Im Sinne der vorliegenden Erfindung sind röntgenamorphe Siliciumdioxidpigmente besonders vorteilhaft, und unter diesen gerade solche des Aerosil®-Typs bevorzugt.

Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974, Aerosil® R976.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% röntgenamorphe Oxidpigmente.

Die nichtröntgenamorphen anorganischen Pigmente liegen erfindungsgemäß vorteilhaft in hydrophober Form vor, d.h., dass sie oberflächlich wasserabweisend behandelt sind. Diese Oberflächenbehandlung kann darin bestehen, dass die Pigmente nach an sich bekannten Verfahren mit einer dünnen hydrophoben Schicht versehen werden.

Eines solcher Verfahren besteht beispielsweise darin, dass die hydrophobe Oberflächenschicht nach einer Reaktion gemäß

$$n\ TiO_2 + m\ (RO)_3Si\text{-}R' \text{ -> } n\ TiO_2\ \text{(oberfl.)}$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0 Gew.-% gewählt, bezogen auf das Gesamtgewicht der Zubereitungen.

Die kosmetischen und dermatologischen Zubereitungen können erfindungsgemäß kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem *Rowe Colour Index, 3. Auflage, Society of Dyers and Colourists, Bradford, England, 1971* entnommen.

| **Chemische oder sonstige Bezeichnung** | **CIN** | **Farbe** |
|---|---|---|
| Pigment Green | 10006 | grün |
| Acid Green 1 | 10020 | grün |
| 2,4-Dinitrohydroxynaphthalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1 -(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |

(fortgesetzt)

| Chemische oder sonstige Bezeichnung | CIN | Farbe |
|---|---|---|
| 4-(2'-Methoxy-5'-sulfosäurediethylamid-1'-phenylazo)-3-hydroxy-5''-chloro-2'',4''-dimethoxy-2-naphthoesäureanilid | 12490 | rot |
| Disperse Yellow 16 | 12700 | gelb |
| 1-(4-Sulfo-1-phenylazo)-4-amino-benzol-5-sulfosäure | 13015 | gelb |
| 2,4-Dihydroxy-azobenzol-4'-sulfosäure | 14270 | orange |
| 2-(2,4-Dimethylphenylazo-5-sulfosäure)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1 ,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1 -(2-Sulfo-4-methyl-5-chlor-1 -phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8-trisulfosäure | 16290 | rot |
| 8-Amino-2 -phenylazo- 1 -naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-pyrazolon-3-carbonsäure | 19140 | gelb |
| Pigment Yellow 16 | 20040 | gelb |
| 2,6-(4'-Sulfo-2'', 4''-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4''-Sulfo-1''-phenylazo)-7'-sulfo-1'-naphthylazo]-1-hydroxy-7-aminonaphthalin-3,6-disulfosäure | 27755 | schwarz |
| 4'-[(4''-Sulfo-1''-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-β-Apo-8'-Carotinaldehyd ($C_{30}$) | 40820 | orange |
| trans-Apo-8'-Carotinsäure ($C_{30}$)-ethylester | 40825 | orange |
| Canthaxanthin | 40850 | orange |

(fortgesetzt)

| Chemische oder sonstige Bezeichnung | CIN | Farbe |
|---|---|---|
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino)-phenyl-(2-sulfophenyl)-methylen-(N-ethyl-N-p-sulfo-benzyl) $\Delta^{2,5}$-cyclohexadienimin | 42090 | blau |
| Acid Green 9 | 42100 | grün |
| Diethyl-di-sulfobenzyl-di-4-amino-2-chlor-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | blau |
| 4'-(N-Dimethyl)-amino-4"-(N-phenyl)-aminonaphtho-N-dimethylfuchsonimmonium | 44045 | blau |
| 2-Hydroxy-3,6-disulfo-4,4'-bis-dimethylaminonaphthofuchsonimmonium | 44090 | grün |
| Acid Red 52 | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidino)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |

(fortgesetzt)

| Chemische oder sonstige Bezeichnung | CIN | Farbe |
|---|---|---|
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268 :1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Manganamimoniumdiphosphat | 77742 | violett |
| Manganphosphat; $Mn_3(PO_4)_2 \cdot 7\ H20$ | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalze, Anthocyane | | rot |
| Aluminium-, Zink-, Magnesium- und Calciumstearat | | weiß |
| Bromthymolblau | | blau |

(fortgesetzt)

| Chemische oder sonstige Bezeichnung | CIN | Farbe |
|---|---|---|
| Bromkresolgrün | | grün |
| Acid Red 195 | | rot |

Es kann ferner günstig sein, als Farbstoff eine oder mehrer Substanzen aus der folgenden Gruppe zu wählen: 2,4-Dihydroxyazobenzol, 1-(2'-Chlor-4'-nitro-1'phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner Gelcrèmes mit einem Gehalt an Perlglanzpigmenten. Bevorzugt sind insbesondere die im folgenden aufgelisteten Arten von Perlglanzpigmenten:

1. Natürliche Perlglanzpigmente, wie z. B.

- "Fischsilber" (Guanin/Hypoxanthin-Mischkristalle aus Fischschuppen) und
- "Perlmutt" (vermahlene Muschelschalen)

2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung / Schichtdicke | Farbe |
|---|---|---|
| **Silberweiße Perlglanzpigmente** | $TiO_2$: 40 - 60 nm | silber |
| **Interferenzpigmente** | $TiO_2$: 60 - 80 nm | gelb |
| | $TiO_2$: 80 - 100 nm | rot |
| | $TiO_2$: 100 - 140 nm | blau |
| | $TiO_2$: 120 - 160 nm | grün |
| **Farbglanzpigmente** | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| **Kombinationspigmente** | $TiO_2$ / $Fe_2O_3$ | Goldtöne |
| | $TiO_2$ / $Cr_2O_3$ | grün |
| | $TiO_2$ / Berliner Blau | tiefblau |

(fortgesetzt)

| Gruppe | Belegung / Schichtdicke | Farbe |
|---|---|---|
| | $TiO_2$ / Carmin | rot |

Besonders bevorzugt sind z.B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit $TiO_2$ und $Fe_2O_3$ beschichtete $SiO_2$-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Perlglanzpigmente, welche unter der Verwendung von $SiO_2$ hergestellt werden. Solche Pigmente, die auch zusätzlich gonichromatische Effekte haben können, sind z. B. unter dem Handelsnamen Sicopearl Fantastico bei der Firma BASF erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihrer Partikelgröße von 40 - 180 µm zusätzlich zu der Farbe einen Glitzereffekt auf.

Besonders vorteilhaft sind ferner auch Effektpigmente, welche unter der Handelsbezeichnung Metasomes Standard / Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α -Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoëharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 0,1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung,

zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Eine gegebenenfalls vorhandene Lipidphase kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Eine Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether, Butylen Glycol Dicaprylat/Dicaprat.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wäßrigen Phase einzusetzen.

Vorteilhaft können die Lichtschutzformulierungen erfindungsgemäß weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 20 Gew.-%, insbesondere 1 bis 15 Gew.-% beträgt, bezogen auf das Gesamtgewicht der Zubereitungen, um kosmetische Zubereitungen zur Verfügung zu stellen, welche die Haut vor dem gesamten Bereich der ultravioletten Strahlung schützen. Sie können auch als Sonnenschutzmittel dienen.

Die weiteren UVB-Filter können öllöslich oder wasserlöslich sein. Vorteilhafte öllösliche UVB-Filtersubstanzen sind z.B.:

- 4-Aminobenzoësäure-Derivate, vorzugsweise 4-(Dimethylamino)-benzoësäure(2-ethylhexyl)ester, 4-(Dimethylamino)benzoësäureamylester;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure

Vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und deren Salze,
- Sulfonsäurederivate von Benzophenon.

Die Liste der genannten UVB-Filter, die zusätzlich im Sinne der vorliegenden Erfindung eingesetzt werden können, soll selbstverständlich nicht limitierend sein.

Es kann auch von Vorteil sein, UVA-Filter einzusetzen, die bisher üblicherweise in kosmetischen Zubereitungen enthalten sind. Es können die für die UVB-Kombination verwendeten Mengen eingesetzt werden.

Ferner kann erfindungsgemäß gegebenenfalls von Vorteil sein, die Zubereitungen mit weiteren UVA- und/oder UVB-Filtern zu versehen, beispielsweise bestimmten Salicylsäurederivaten wie

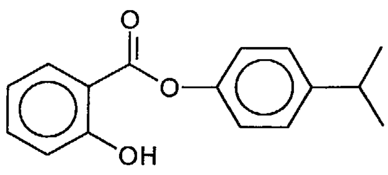

(4-Isopropylbenzylsalicylat),

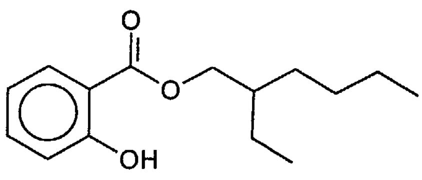

(2-Ethylhexylsalicylat, Octylsalicylat),

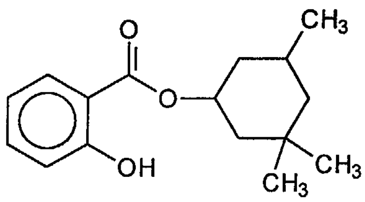

(Homomenthylsalicylat).

Die Gesamtmenge an einem oder mehreren Salicylsäurederivaten in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 3 Gew.-% zu wählen. Wenn Homomenthylsalicylat gewählt wird, ist es von Vorteil, dessen Gesamtmenge aus dem Bereich von 0,1 - 10,0 Gew.-%, bevorzugt 0,5 - 5,0 Gew.-% zu wählen.

Noch eine weiterere erfindungsgemäß vorteilhaft zu verwendende zusätzliche Lichtschutzfiltersubstanz ist das Ethylhexyl-2-cyano-3,3-diphenylacrylat (Octocrylen), welches von BASF unter der Bezeichnung UVINUL® N 539 erhältlich ist und sich durch folgende Struktur auszeichnet:

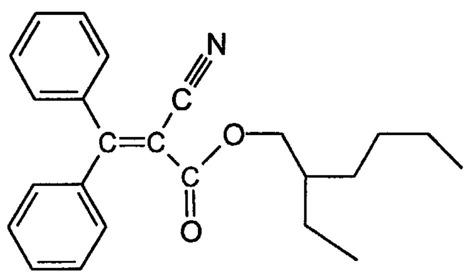

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispiele:**

**1. O/W Emulsionen**

| | 1 | 2 | 3 | 4 | 5 | 6 | 7 |
|---|---|---|---|---|---|---|---|
| Dihydroxyaceton | 4,0 | 6,0 | 5,0 | 4,0 | 3,0 | 5,0 | 2,0 |
| Glycerinmonostearat SE | | | 1,5 | | | 0,5 | 4,0 |
| Glycerylstearatcitrat | 2,0 | | | | | 3,0 | |
| PEG-40 Rizinus Öl | | 2,0 | | | | | |
| Glycerylstearat | | | | 5,0 | | | |
| PEG-30 Stearat | | | | 1,5 | | | |
| Natriumstearat | | 0,5 | | | | | |
| Stearinsäure | | | | | 2,0 | | 2,0 |
| PEG-40 Stearat | | | | | | 0,5 | |

(fortgesetzt)

| | 1 | 2 | 3 | 4 | 5 | 6 | 7 |
|---|---|---|---|---|---|---|---|
| Cetylphosphat | 1,0 | | | | 2,5 | | |
| Cetearylalkohol | | 1,0 | 1,0 | | | | |
| Cetylalkohol | | | 2,0 | | 2,0 | 2,5 | |
| Polyglyceryl-3 Methylglucosedistearat | | | | | 3,5 | | |
| Glyceryllanolat | 0,5 | | | | 0,5 | | |
| Lanolinalkohol | | | | | 0,5 | | |
| Myristylalkohol | | | | | 1,0 | | |
| Natriumcetearylsulfat | | 1,0 | 1,0 | | | | |
| Bis-Ethylhexyloxyphenol methoxyphenyltriazin | 0,5 | 1,5 | 0,75 | 1,7 | 2,0 | 0,7 | 1,5 |
| Dinatriumphenyl dibenzimidazoltetrasulfonat | 1,0 | 2,0 | | 1,5 | | | 0,5 |
| Methylen-bis-benztriazolyl tetramethylbutylphenol | 3,0 | | 4,0 | 1,0 | 2,0 | 3,0 | |
| Drometrizoltrisiloxan | | | | 1,0 | 2,0 | 0,5 | 1,0 |
| Octocrylen | | 5,0 | | | 3,0 | | 2,5 |
| Ethylhexylmethoxycinnamat | | | 2,0 | 4,0 | | | |
| Ethylhexylsalicylat | 4,0 | | 1,5 | | | | |
| Diethylhexylbutamidotriazon | 2,0 | 1,5 | 2,0 | | | 3,0 | |
| Ethylhexyltriazon | | | | 2,0 | 1,0 | | 1,0 |
| Titandioxid | | | | 0,50 | | | 0,50 |
| Octyldodecanol | 5,00 | | 3,00 | | 3,50 | | 3,00 |
| C12-15 Alkylbenzoat | | | 5,00 | | | | |
| Dicaprylylether | | 5,00 | | | | 5,00 | |
| Capryl-/Caprintriglycerid | | | 2,00 | | 4,00 | | |
| Dicaprylylcarbonat | | | | | | | |
| Dimethicon | | | | 3,00 | | 2,00 | |
| Cyclomethicon | | | 3,00 | | | | |
| Shea Butter | | 0,50 | | | 1,00 | | |
| Trinatrium EDTA | 1,00 | | | | | 1,00 | 0,50 |
| Glycerin | | | 3,00 | | 3,00 | | 3,00 |
| Xanthan Gummi | | | 0,50 | | 1,00 | | |
| Natrium Carbomer | | 0,50 | | | | 0,50 | |
| Vitamin E Acetat | 0,50 | | 0,50 | | 0,50 | | |
| Jodopropynylbutylcarbamat | 0,10 | | | 0,10 | | 0,10 | |
| Methylparaben | 0,30 | | 0,25 | 0,03 | 0,25 | 0,25 | 0,30 |
| Phenoxyethanol | | 0,50 | 0,25 | | 0,25 | 0,25 | |
| Ethanol | | | 5,00 | | 3,00 | | 3,50 |

(fortgesetzt)

| | 1 | 2 | 3 | 4 | 5 | 6 | 7 |
|---|---|---|---|---|---|---|---|
| Farbstoffe (öl- und/oder wasserlöslich) | 0,01 | | | 0,03 | 0,05 | | |
| Trinatrium EDTA | 0,4 | 0,25 | | 0,3 | | 0,4 | 0,25 |
| Citrat-Puffer | q.s. | q.s. | q.s. | q.s. | q.s. | q.s. | q.s. |
| Parfüm | 0,3 | 0,2 | 0,45 | 0,3 | 0,2 | | 0,3 |
| Wasser | ad. 100 | ad. 100 | ad. 100 | ad. 100 | ad. 100 | ad. 100 | ad. 100 |

**2. Hydrodispersionen**

| | 1 | 2 | 3 | 4 | 5 |
|---|---|---|---|---|---|
| Dihydroxyaceton | 3,0 | 7,0 | 5,0 | 4,0 | 5,0 |
| Ceteareth-20 | | 0,5 | | | 1,5 |
| Cetylalkohol | | | 0,75 | | |
| Natrium Carbomer | 0,4 | 0,2 | | | |
| Acrylat/C10-30 Alkyl Acrylat Crosspolymer | 0,15 | | 0,4 | | 0,2 |
| Xanthan Gummi | | 0,5 | | 0,8 | 0,4 |
| Bis-Ethylhexyloxyphenol Methoxyphenyltriazin | 0,5 | 2,0 | 1,0 | 4,0 | 5,0 |
| Dinatriumphenyldibenzimidazol tetrasulfonat | 0,5 | 1,0 | | 2,0 | |
| Methylen-bis-benztriazolyl tetramethylbutylphenol | | 2,0 | | 1,0 | 4,0 |
| Drometrizoltrisiloxan | 1,5 | | 1,0 | 0,2 | |
| Octocrylen | | 4,0 | 2,0 | | |
| Ethylhexylmethoxycinnamat | 2,0 | | | 7,0 | |
| Ethylhexylsalicylat | | 4,5 | 4,0 | | |
| Diethylhexylbutamidotriazon | 3,0 | | | | 1,5 |
| Ethylhexyltriazon | 2,0 | | 1,5 | | 3,0 |
| Titandioxid | | 1,0 | | 2,0 | 0,5 |
| Zinkoxid | | 0,5 | 0,5 | | |
| C12-15 Alkylbenzoat | 5,0 | | | | 9,0 |
| Dicaprylylether | | 1,0 | 2,0 | | |
| Butylenglycoldicaprylat/-dicaprat | 3,0 | | | | 1,0 |
| Dicaprylylcarbonat | 2,0 | 4,0 | | | |
| Dimethicon | | | 1,0 | 2,0 | |
| Phenyltrimethicon | | | 0,5 | | 2,0 |
| Shea Butter | 2,0 | | | | |
| PVP Hexadecen Copolymer | | 0,5 | | 1,0 | |
| Octoxyglycerin | 0,3 | 0,3 | | 0,5 | |
| Glycerin | 5 | 7,5 | 10 | | 7,5 |
| Glycin Soja | | | | 1,5 | |

(fortgesetzt)

| | 1 | 2 | 3 | 4 | 5 |
|---|---|---|---|---|---|
| Vitamin E Acetat | 0,5 | 1,5 | 0,3 | | 1,0 |
| Polyurethan | | 1,0 | | 1,0 | |
| DMDM Hydantoin | | 0,1 | | | |
| Konkaben LMB ® | | | | | 0,2 |
| Methylparaben | | | 0,3 | | 0,3 |
| Phenoxyethanol | | 1,0 | 0,4 | | |
| Ethanol | 4,0 | | 2,0 | 5,0 | |
| Parfüm | 0,4 | | 0,2 | | 0,2 |
| Trinatrium EDTA | | 0,2 | 0,3 | | 0,4 |
| Farbstoff | 0,05 | | | 0,1 | |
| Citrat-Puffer | q.s. | q.s. | q.s. | q.s. | q.s. |
| Wasser | ad. 100 | ad. 100 | ad. 100 | ad. 100 | ad. 100 |

**3. W/O Emulsionen**

| | 1 | 2 | 3 | 4 | 5 |
|---|---|---|---|---|---|
| Dihydroxyaceton | 3,0 | 6,0 | 5,0 | 4,0 | 5,0 |
| Cetyldimethicon Copolyol | 2,5 | 0,75 | | 3,5 | |
| Polyglyceryl-2-dipolyhydroxystearat | 1,0 | | 3,0 | | 3 |
| PEG-30-dipolyhydroxystearat | | 2,0 | | | 2,5 |
| Bis-Ethylhexyloxyphenol Methoxyphenyltriazin | 0,5 | 1,0 | 4,0 | 0,5 | 2,5 |
| Dinatriumphenyldibenzimidazol tetrasulfonat | 1,5 | 1,0 | | | 2,0 |
| Methylen-bis-benztriazolyl tetramethylbutylphenol | | | 4,0 | | 4,0 |
| Drometrizoltrisiloxan | | 1,0 | | 2,5 | |
| Octocrylen | | 4,0 | | 7,0 | 5,0 |
| Ethylhexylmethoxycinnamat | 7,0 | 4,0 | | | |
| Ethylhexylsalicylat | | | 2,0 | | |
| Diethylhexylbutamidotriazon | 2,0 | 3,0 | | 2,5 | |
| Ethylhexyltriazon | 2,0 | | 3,5 | | |
| Titandioxid | | 2,0 | 3,0 | | |
| Zinkoxid | 0,5 | | | | 1,5 |
| Mineralöl | 6,0 | 5,5 | 8,5 | | |
| C12-15 Alkylbenzoate | 2,0 | | 3,0 | | |
| Dicaprylylether | | 1,5 | | | 4,0 |
| Butylenglycoldicaprylat/-dicaprat | 7,5 | 6,0 | | | 5,0 |
| Dicaprylylcarbonat | | | | 2,0 | |
| Dimethicon | | 2,0 | | 6,0 | |

(fortgesetzt)

| | 1 | 2 | 3 | 4 | 5 |
|---|---|---|---|---|---|
| Cyclomethicon | 1,5 | | | 5,0 | |
| Shea Butter | 0,75 | | 2,0 | | |
| PVP Hexadecen Copolymer | | 0,5 | | 0,75 | |
| Octoxyglycerin | 0,3 | | | | 0,75 |
| Glycerin | 10 | | 7,5 | 5,0 | |
| Butylenglycol | | 7,5 | | 4,0 | 10 |
| Glycin Soja | 1,0 | 1,5 | | | 0,5 |
| Magnesiumsulfat | 0,7 | | 0,8 | 1,2 | |
| Magnesiumchlorid | | 0,5 | | | 0,8 |
| Vitamin E Acetat | 0,5 | 1,0 | 0,75 | 0,5 | |
| DMDM Hydantoin | | | 0,1 | | |
| Methylparaben | | 0,4 | 0,3 | | 0,3 |
| Phenoxyethanol | | 1,0 | | 0,5 | |
| Ethanol | 2,0 | | 4,0 | 2,5 | |
| Trinatrium EDTA | | 0,7 | 0,3 | | 0,5 |
| Parfüm | 030 | | 0,40 | 0,20 | |
| Citrat-Puffer | q.s. | q.s. | q.s. | q.s. | q.s. |
| Wasser | ad. 100 | ad. 100 | ad. 100 | ad. 100 | ad. 100 |

**4. PIT - Sprays**

| | 1 | 2 | 3 | 4 | 5 |
|---|---|---|---|---|---|
| Dihydroxyaceton | 3,0 | 6,0 | 5,0 | 4,0 | 5,0 |
| Glycerinmonostearat SE | 4,0 | | | 3,5 | |
| Ceteareth-12 | | 0,5 | | 0,2 | |
| Ceteareth-20 | 3,0 | | | 2,5 | |
| Ceteareth-30 | | 2,5 | | | |
| Isoceteth-20 | | | 5,4 | | 4,0 |
| Glycerylisostearat | | 4,5 | 3,5 | | 5,0 |
| Stearylalkohol | | 2,0 | | 0,75 | |
| Cetylalkohol | 2,0 | | 2,5 | | 1,5 |
| Bis-Ethylhexyloxyphenol methoxyphenyltriazin | 0,75 | 3,0 | 4,0 | 1,0 | 1,7 |
| Dinatriumphenyldibenzimidazol tetrasulfonat | 2,0 | | 2,5 | 0,5 | |
| Methylen-bis-benztriazolyl tetramethylbutylphenol | | 4,0 | | 2,5 | |
| Drometrizoltrisiloxan | 0,7 | 1,0 | | 2,0 | 1,5 |
| Octocrylen | 2,0 | | 5,0 | | 4,0 |
| Ethylhexylmethoxycinnamat | | 4,0 | 2,5 | 7,5 | |

(fortgesetzt)

| | 1 | 2 | 3 | 4 | 5 |
|---|---|---|---|---|---|
| Ethylhexylsalicylat | | 2,5 | 3,0 | | |
| Diethylhexylbutamidotriazon | 1,0 | | | 1,5 | |
| Ethylhexyltriazon | 2,0 | | 1,0 | | |
| Phenylbenzmidazolsulfonsäure | 1,0 | 2,0 | | | 2,0 |
| C12-15 Alkylbenzoat | 2,0 | | 2,0 | 2,0 | |
| Dicaprylylether | | 1,0 | | | |
| Butylenglycoldicaprylat/-dicaprat | | | 2,0 | 3,0 | |
| Dicaprylylcarbonat | 4,0 | | | 0,5 | 5,0 |
| Dimethicon | | 0,75 | | | |
| Phenyltrimethicon | 1,5 | | 1,0 | 0,75 | |
| Shea Butter | | 2,0 | 2,0 | | |
| PVP Hexadecen Copolymer | 0,5 | | 1,0 | | 1,0 |
| Tricontanyl PVP | | | | 0,8 | 0,5 |
| Glycerin | 10 | | 7,5 | 5,0 | 5,0 |
| Butylengylcol | | 10 | 3,5 | | |
| Vitamin E Acetat | 0,5 | 1,0 | | 0,5 | |
| DMDM Hydantoin | | | | | 0,1 |
| Konkaben LMB ® | | | 0,18 | 0,2 | |
| Methylparaben | 0,3 | | 0,2 | | |
| Phenoxyethanol | | 1,0 | 0,5 | 1,0 | 0,0 |
| Ethanol | 3,0 | 2,0 | | | |
| Parfüm | 0,2 | 0,2 | 0,5 | 0,3 | 0,4 |
| Trinatrium EDTA | 0,25 | 0,5 | | 0,3 | 0,1 |
| Farbstoff | 0,01 | | 0,03 | 0,15 | |
| Citrat - Puffer | q.s. | q.s. | q.s. | q.s. | q.s. |
| Wasser | ad. 100 | ad. 100 | ad. 100 | ad. 100 | ad. 100 |

**5. Feststoffstabilisierte Emulsionen**

| | 1 | 2 | 3 | 4 | 5 |
|---|---|---|---|---|---|
| Dihydroxyaceton | 5,0 | 4,0 | 5,0 | 7,0 | 5,0 |
| Mineralöl | 15 | | 7,5 | 9,0 | |
| Octyldodecanol | | 9,0 | 6,0 | | 10,0 |
| Capryl-/Caprintriglycerid | 5,5 | 5,0 | | | |
| C12-15- Alkylbenzoat | | | 4,0 | 7,0 | |
| Butylenglycoldicaprylat/dicaprat | 7,0 | 9,0 | | 5,0 | 9,0 |
| Dicaprylylether | | 3,0 | 2,5 | | |

(fortgesetzt)

| | 1 | 2 | 3 | 4 | 5 |
|---|---|---|---|---|---|
| Dicaprylylcarbonat | 4,5 | | 5,0 | | 3,0 |
| Hydroxyoctacosanyl hydroxystearat | 2,0 | | 1,0 | 1,5 | 2,0 |
| Disteardimoniumhectorit | 1,5 | 1,75 | 3,5 | 2,0 | 0,7 |
| Vaseline | | 2,0 | | 0,5 | |
| Hydroxypropylmethylcellulose | 0,2 | | 0,15 | | |
| Dimethicon | | 1,5 | | | 4,0 |
| Bis-Ethylhexyloxyphenol methoxyphenyltriazin | 0,5 | 4,0 | 1,0 | 3,5 | 2,0 |
| Dinatriumphenyldibenzimidazol tetrasulfonat | 2,0 | 2,5 | 1,5 | | 1,5 |
| Methylen-bis-benztriazolyl tetramethylbutylphenol | | 4,0 | 1 | 3,0 | |
| Drometrizoltrisiloxan | | | 1,5 | 2,0 | 0,5 |
| Octocrylen | 5,0 | | 2,5 | 7.0 | |
| Ethylhexylmethoxycinnamat | | 5,0 | | | 5,0 |
| Ethylhexylsalicylat | 0,5 | | | | |
| Diethylhexylbutamidotriazon | | 1,0 | 2,5 | | 0,5 |
| Ethylhexyltriazon | | 3,0 | | 1,0 | 2,5 |
| Phenylbenzmidazolsulfonsäure | | 1,5 | | | |
| Titandioxid + Aluminumoxid + Simethicon + Wasser | 4,0 | 3,5 | | 2,5 | |
| Titandioxid + Trimethoxycaprylylsilan | | | 3,0 | 2,0 | 3,0 |
| Zinkoxid | 4,0 | | 2,5 | | 3,0 |
| Siliziumdimethylsilylat | | 1,0 | | 0,7 | |
| Bornitrid | | 2,0 | 3,5 | | |
| Stärke/-Natriummetaphosphat-Polymer | 1,5 | | | | 3,0 |
| Tapioca Stärke | | 1,0 | 0,7 | | |
| Natriumchlorid | 1,0 | 1,5 | | 0,7 | |
| Magnesiumsulfat | | | 1,0 | | 0,5 |
| Glycerin | 5,0 | 10,0 | 7,5 | 3,5 | 5,0 |
| Trinatrium EDTA | | 0,4 | 0,25 | 0,3 | 0,7 |
| Methylparaben | 0,3 | | | 0,3 | 0,3 |
| Propylparaben | 0,1 | | 0,3 | 0,2 | |
| Phenoxyethanol | 0,5 | | | | 1,0 |
| Hexamidindiisethionat | | 0,01 | | | |
| Diazolidinylharnstoff | | | 0,02 | | |
| Ethanol | 2,0 | | | 5,0 | |
| Farbstoff | | 0,1 | | 0,02 | 0,04 |
| Parfüm | q.s. | q.s. | q.s. | q.s. | q.s. |
| Wasser | Ad 100 | Ad 100 | Ad 100 | Ad 100 | Ad 100 |

**Patentansprüche**

1. Kosmetische Formulierungen, die mindestens einen UV-Filter aus der Gruppe der Triazine, mindestens eine Selbstbräunungssubstanz enthalten und frei von Dibenzoylmethan- und Campherderivaten sind.

2. Kosmetische Formulierungen nach Anspruch 1, welche als Selbstbräunungssubstanz 1,3-Dihydroxyaceton enthalten.

3. Kosmetische Formulierungen nach Anspruch 1 und 2, welche als Selbstbräunungssubstanz 1,3-Dihydroxyaceton in einer Konzentration von 0,1 bis 10 Gewichts-% enthalten.

4. Kosmetische Formulierungen nach Anspruch 1 bis 3, welche die UV-Filter aus der Gruppe der Triazine in einer Konzentration von 0,01 bis 15 Gewichts-%, insbesondere in einer Konzentration von 0,1 bis 10 Gewichts-% enthalten.

5. Kosmetische Formulierungen nach Anspruch 1 bis 4, die als UV-Filter 2,4-Bis-{[4-(2-ethyl-hexyloxy)-2-hydroxy]-phenyl}-6-(4-methoxyphenyl)-1,3,5-triazin (INCI: Aniso Triazin) und/oder Dioctylbutylamidotriazon (INCI: Dioctylbutamidotriazone) und/oder 4,4',4''-(1,3,5-Triazin-2,4,6-triyltriimino)-tris-benzoesäure-tris(2-ethylhexylester) (synonym: 2,4,6-Tris-[anilino-(p-carbo-2'-ethyl-1'-hexyloxy)]-1,3,5-triazin, INCI: Ethylhexyl Triazin) enthalten.

6. Kosmetische Formulierungen nach Anspruch 1 bis 5, welche weitere UV-Filter enthalten.

7. Kosmetische Formulierungen nach Anspruch 1 bis 6, welche anorganische Pigmente, insbesondere Zinkoxid und / oder Titandioxid enthalten

8. Kosmetische Formulierungen nach Anspruch 1 bis 7, bei denen das Verhältnis von UV-Filter und Selbstbräuner 0,1:10 bis 10:0,1 beträgt.

9. Kosmetische Formulierungen nach Anspruch 1 bis 8, welche lösliche und/oder pigmentäre Farbstoffe enthalten.

10. Kosmetische Formulierungen nach Anspruch 1 bis 9, welche photostabil sind.

11. Verwendung der kosmetischen Formulierungen nach Anspruch 1 bis 10 als Sonnenschutz- und/oder Selbstbräunungsmittel.